# EUROPEAN PATENT SPECIFICATION

(11) **EP 1 337 849 B1**
(45) Date of publication and mention of the grant of the patent: **18.11.2009**
(21) Application number: 01995263.9
(22) Date of filing: 29.11.2001
(51) Int. Cl.: G01N 33/569

(54) **METHODS AND COMPOSITIONS FOR SORTING AND/OR DETERMINING MICROORGANISMS**
VERFAHREN UND ZUSAMMENSETZUNGEN ZUM AUSSORTIEREN UND/ODER NACHWEIS VON MIKROORGANISMEN
PROCEDES ET COMPOSITIONS DE TRI ET/OU DE DETERMINATION D'ORGANISMES

(30) Priority: 30.11.2000 US 250930 P
(43) Date of publication of application: 27.08.2003
(73) Proprietor: Boston Probes, Inc., Bedford, MA 01730 (US)
(72) Inventor: COULL, James, M., Westford, MA 01886 (US); STENDER, Henrik, 2820 Gentofte (DK); GILDEA, Brian, D., Billerica, MA 01821 (US)
(74) Representative: Smart, Peter John
(86) International application number: PCT/US2001/044728
(87) International publication number: WO 2002/044733

(56) References cited:
- WO-A-02/42736
- US-A- 4 591 570
- IANNELLI D ET AL: "Simultaneous detection of cucumber mosaic virus, tomato mosaic virus and potato virus Y by flow cytometry." JOURNAL OF VIROLOGICAL METHODS, vol. 69, no. 1-2, December 1997 (1997-12), pages 137-145, XP001117743 ISSN: 0166-0934
- KULAGA H ET AL: "Identification of pathogenic agents via microsphere-based immunoassays on a flow cytometer." CYTOMETRY, no. SUPPL. 9, 1998, pages 55-56, XP001118576 XIX International Congress of the International Society for Analytical Cytology;Colorado Springs, Colorado, USA; February 28-March 5, 1998 ISSN: 0196-4763
- LUK J M C ET AL: "RAPID AND SENSITIVE DETECTION OF SALMONELLA O 6 7 BY IMMUNOMAGNETIC MONOCLONAL ANTIBODY-BASED ASSAYS" JOURNAL OF IMMUNOLOGICAL METHODS, vol. 137, no. 1, 1991, pages 1-8, XP000566449 ISSN: 0022-1759
- DATABASE BIOSIS [Online] BIOSCIENCES INFORMATION SERVICE, PHILADELPHIA, PA, US; 1996 WALLNER GUENTER VVO STEINMETZ ET AL: "Combination of rRNA-targeted hybridization probes and immuno-probes for the identification of bacteria by flow cytometry." Database accession no. PREV199799404129 XP002218123 & SYSTEMATIC AND APPLIED MICROBIOLOGY, vol. 19, no. 4, 1996, pages 569-576, ISSN: 0723-2020
- ALVAREZ-BARRIENTOS ALBERTO ET AL: "Applications of flow cytometry to clinical microbiology." CLINICAL MICROBIOLOGY REVIEWS, vol. 13, no. 2, April 2000 (2000-04), pages 167-195, XP002218122 ISSN: 0893-8512
- FULTON R J ET AL: "Advanced multiplexd analysis with the FlowMetrix(TM) system" CLINICAL CHEMISTRY, AMERICAN ASSOCIATION FOR CLINICAL CHEMISTRY. WINSTON, US, vol. 43, no. 9, September 1997 (1997-09), pages 1749-1756, XP002142645 ISSN: 0009-9147

## Description

### Background of the Invention

### 1. Technical Field

This invention is related to the field of combined probe-based and binding partner-based detection, analysis and/or quantitation of organisms and/or cells.

### 2. Background Art

Nucleic acid hybridization is a fundamental process in molecular biology. Probe-based assays are useful in the detection, quantitation and/or analysis of nucleic acids. Nucleic acid probes have long been used to analyze samples for the presence of nucleic acid from bacteria, fungi, virus or other organisms and are also useful in examining genetically-based disease states or clinical conditions of interest. Nonetheless, nucleic acid probe-based assays have been slow to achieve commercial success. This lack of commercial success is, at least partially, the result of difficulties associated with specificity, sensitivity and/or reliability.

Despite its name, Peptide Nucleic Acid (PNA) is neither a peptide, a nucleic acid nor is it an acid. Peptide Nucleic Add (PNA) is a non-naturally occurring polyamide that can hybridize to nucleic acid (DNA and RNA) with sequence specificity (See: United States Patent No. 5,539,082 and Egholm et al., Nature 365: 566-568 (1993)). In point of fact, PNA has been consistently characterized as a nucleic acid mimic rather than a nucleic acid analog since it is not derived from nucleic acid or its component nucleotides or nucleosides as well as comprises unique properties (See: Nielsen, P.E., Acc. Chem. Res. 32: 624-630 (1999)). Being a non-naturally occurring molecule, unmodified PNA is not known to be a substrate for the enzymes that are known to degrade peptides or nucleic acids. Therefore, PNA should be stable in biological samples, as well as have a long shelf-life. Unlike nucleic acid hybridization, which is very dependent on ionic strength, the hybridization of a PNA with a nucleic acid is fairly independent of ionic strength and is favored at low ionic strength, conditions that strongly disfavor the hybridization of nucleic acid to nucleic acid (Egholm et al., Nature, at p. 567). Because of their unique properties, it is clear that PNA is not the equivalent of a nucleic acid in either structure or function. Consequently, PNA probes need to be evaluated for performance and optimization to thereby confirm whether they can be used to specifically and reliably detect a particular nucleic acid target sequence, particularly when the target sequence exists in a complex sample such as a cell, tissue or organism.

Like nucleic acid probes, antibodies have been used to examine samples for the purpose of determining organisms. Labeled nucleic acid probes used in combination with labeled antibodies have also been used to identify bacteria by flow cytometric analysis. (See: Wallner et al., System. Appl. Mircobiol., 19: 569-576 (1996)). Importantly, Wallner et al. analyzed the bacteria as stained by the labeled nucleic acid probes and labeled antibodies but did not immobilize the bacteria to a particle, bead or other solid carrier for analysis.

Luminex (Austin, Texas) has recently introduced coded beaded supports and an instrument suitable for determining the coded beaded supports as well as one or more reporter moieties bound thereto. (See: Luminex Product Literature) The coded beaded supports incorporate a proprietary, precision process to internally dye same-sized polystyrene microspheres with two fluorophores. *Id*. Using precise ratios of the two fluorophores, Luminex has created 100 different coded microsphere sets wherein each set is distinguished based on its internal dye ratio using the Luminex 100^{™}. *Id.* In addition to determining the code of each bead, the Luminex 100^{™} can also determine a reporter moiety on the beads with highly accurate quantitation. *Id*.

By providing coded beaded supports having surface carboxyl groups or Lumavidin^{™}_{,} Luminex proposes that their coded beaded supports and instruments can be adapted for applications involving Molecular Biology, Immunoassays, Enzymatic Assays & Reporter-Ligand Assays, depending on the nature of the immobilized ligands: *Id.* Importantly, Luminex does not teach or suggest the integration of specific analyte capture with specific analyte staining as a means to achieve improved discrimination in the assay or certainty of the result and in particular, Luminex does not appear to teach or suggest that organisms of any kind can be bound to and determined using their beads and/or instrument. In fact, Applicants are not aware of any example of using a specific binding partner to immobilize, for determination, specifically stained organisms of interest to a solid carrier.

### Disclosure Of The Invention

### 1. Summary:

This invention is generally directed to methods and compositions as defined in the claims that pertain to the use of molecular probes, for the selective staining of organisms or cells, in combination with the selective capture of organisms or cells using binding partners. The methods and compositions of this invention can be used for sorting and/or determining an organism or organisms of interest. When sorting is chosen, sorting can be performed, for example, either by the use of coded beaded supports or by the use of an array. Because selectivity can be affected at two very different levels of molecular recognition, the methods and compositions of this invention provide for enhanced assay discrimination and/or enhanced certainty of the result.

The utility of the methods and compositions of this invention can be further enhanced by the use of independently detectable molecular probes that facilitate the multiplexing of the staining process. Multiplexing of the methods and compositions is also facilitated at the immobilization step of the process by, for examples, the use of different binding partners that are used for the selective immobilization of different organisms of interest. Multiplexing an assay at the immobilization step can also be facilitated by the use of coded beaded supports wherein the "code" for the different beads is associated with the sample source or with another parameter of interest. Hence, the methods and compositions of this invention facilitate a broad scope of flexibility of analysis in a way that overcomes numerous limitations of the prior art

### 2. Detailed Description Of The Preferred Embodiments Of The Invention:

### I. Definitions:

For the purposes of interpreting of this specification the following definitions shall apply and whenever appropriate, terms used in the singular shall also include the plural and vice versa.
a. As used herein, a "nucleobase" means those naturally occurring and those non-naturally occurring heterocyclic moieties commonly known to those who utilize nucleic acid technology or utilize peptide nucleic acid technology to thereby generate polymers that can sequence specifically bind to nucleic acids.
b. As used herein, a "nucleobase sequence" means any segment of a polymer that comprises nucleobase-contairung subunits. Non-limiting examples of suitable polymers or polymers_segments include oligodeoxynucleotides (e.g. DNA), oligoribonucleotides (e.g. RNA), peptide nucleic acid (PNA), nucleic acid analogs, nucleic acid mimics, and/or chimeras.
c. As used herein, a "target sequence" is the nucleobase sequence of a nucleic acid that is found in an organism of interest and to which a molecular probe is designed to hybridize sequence specifically thereto.
d. As used herein, a "nucleic acid" is a nucleobase sequence-containing polymer, or polymer segment, having a backbone formed from nucleotides, or analogs thereof.
e. As used herein, a "non-nucleic acid" is a nucleobase sequence containing polymer, or polymer segment, having a backbone formed from subunits that are not nucleotides, or analogs thereof. Peptide nucleic acids are a preferred non-nucleic acid polymer.
f. As used herein, the term "probe" or "molecular probe" means a nucleic acid or non-nucleic acid polymer (e.g. a DNA, RNA, PNA, nucleic acid analogs, nucleic acid mimics, chimera or linked polymer) having a probing nucleobase sequence that is designed to sequence specifically hybridize to a target sequence of a target molecule of an organism of interest.
g. As used herein, a "detectable molecular probe" is a probe or molecular probe that is detectable by instrument or method. For the avoidance of doubt, a "detectable molecular probe" need not be directly labeled with a detectable moiety (See: the subsection entitled: "Unlabeled Molecular Probes", below for a discussion of determining unlabeled molecular probes).
h. As used herein, the term "antibody" means an antibody or antibody fragment that is capable of participating in an antibody/antigen binding interaction.
i. As used herein, the term "detectable antibody" means an antibody or antibody fragment that is detectable by instrument or method. For the avoidance of doubt, a detectable antibody need not be directly labeled with a detectable moiety since, for example, the antibody may be detected using a secondary antibody that is labeled with a detectable moiety.
j. As used herein, "stained" means that individual organisms are directly or indirectly marked with a detectable moiety as a result of the sequence specific hybridization of one or more detectable molecular probes to a target sequence within the organism.
k. As used herein, the term "peptide nucleic acid" or "PNA" means any oligomer, polymer, linked polymer or chimeric oligomer, comprising two or more PNA subunits (residues), including any of the polymers referred to or claimed as peptide nucleic acids in United States Patent Nos. 5,539,082, 5,527,675, 5,623,049, 5,714,331, 5,718,262, 5,736,336, 5,773,571, 5,766,855, 5,786,461, 5,837,459, 5,891,625, 5,972,610, 5,986,053 and 6,107,470; all of which are herein incorporated by reference. The term "peptide nucleic acid" or "PNA" shall also apply to polymers comprising two or more subunits of those nucleic acid mimics described in the following publications: Lagriffoul et al., Bioorganic & Medicinal Chemistry Lefters, 4: 1081-1082 (1994); Petersen et al., Bioorganic & Medicinal Chemistry Letters, 6: 793-796 (1996); Diderichsen et al, Tett: Lett. 37: 475-478 (1996); Fujii et al., Bioorg. Med. Chem. Lett. 7: 637-627 (1997); Jordan et al., Bioorg. Med. Chem. Lett. 7: 687-690 (1997); Krotz et al., Tett. Lett: 36: 6941-6944 (1995); Lagriffoul et al., Bioorg. Med. Chem. Lett. 4: 1081-1082 (1994); Diederichsen, U., Bioorganic & Medicinal Chemistry Letters, 7: 1743-1746 (1997); Lowe et al., J. Chem. Soc. Perkin Trans. 1, (1997) 1: 539-546; Lowe et al., J. Chem. Soc. Perkin Trans. 11: 547-554 (1997); Lowe et al., J. Chem. Soc. Perkin Trans. 11:5 55-560 (1997); Howarth et al., J. Org. Chem. 62: 5441-5450 (1997); Altmann, K-H et al., Bioorganic & Medicinal Chemistry Letters, 7: 1119-1122 (1997); Diederichsen, U., Bioorganic & Med. Chem. Lett., 8: 165-168 (1998); Diederichsen et aL, Angew. Chem. Int. Ed., 37: 302-305 (1998); Cantin et al., Tett. Lett., 38: 4211-4214 (1997); Ciapetti et al., Tetrahedron, 53: 1167-1176 (1997); Lagriffoule et al., Chem. Eur. J., 3: 912-919 (1997); and the Peptide-Based Nucleic Acid Mimics (PENAMs) of Shah et al. as disclosed in WO96/04000.
   In preferred embodiments, a PNA is a polymer comprising two or more subunits of the formula: 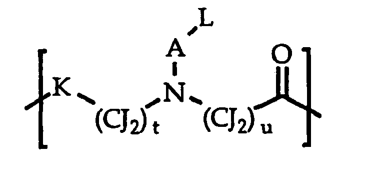 wherein, each J is the same or different and is selected from the group consisting of H, R¹, OR¹, SR¹, NHR¹, NR¹₂ F, Cl, Br and I. Each K is the same or different and is selected from the group consisting of O, S, NH and NR¹. Each R¹ is the same or different and is an alkyl group having one to five carbon atoms that may optionally contain a heteroatom or a substituted or unsubstituted aryl group. Each A is selected from the group consisting of a single bond, a group of the formula; -(CJ₂)₈- and a group of the formula; -(CJ₂)₈C(O)-, wherein, J is defined above and each s is a whole number from one to five. Each t is 1 or 2 and each u is 1 or 2. Each L is the same or different and is independently selected from the group consisting of J, adenine, cytosine, guanine, thymine, uridine, 5-methylcytosine, 2-aminopurine, 2-amino-6-chloropurine, 2,6-diaminopurine, hypoxanthine, pseudoisocytosine, 2-thiouracil, 2-thiothymidine, other naturally occurring nucleobase analogs, other non-naturally occurring nucleobases, substituted and unsubstituted aromatic moieties, biotin, fluorescein and dabcyl. In the most preferred embodiment, a PNA subunits consists of a naturally occurring or non-naturally occurring nucleobase attached to the aza nitrogen of the N-[2-(aminoethyl)]glycine backbone through a methylene carbonyl linkage.
l. As used herein, the terms "label" and "detectable moiety" are interchangeable and refer to moieties that can be attached to a molecular probe, antibody or antibody fragment to thereby render the molecular probe, antibody or antibody fragment detectable by an instrument or method.
m. Asnased herein, the term "chimera" or "chimeric oligomer" means a polymer comprising two or more linked subunits that are selected from different classes of subunits. For example, a PNA/DNA chimera would comprise at least two PNA subunits linked to at least one 2'-deoxyribonucleic acid subunit (For exemplary methods and compositions related to PNA/DNA chimera preparation See: WO96/40709). Exemplary component subunits of the chimera are selected from the group consisting of PNA subunits, naturally and non naturally occurring amino acid subunits, DNA subunits, RNA subunits and subunits of analogues or mimics of nucleic acids.
n. As used herein, the term "linked polymer" means a polymer comprising two or more polymer segments that are linked by a linker. The polymer segments that are linked to form the linked polymer are selected from the group consisting of an oligodeoxynucleotide (DNA), an oligoribonudeotide (RNA), a peptide, a polyamide, a peptide nucleic acid (PNA) and a chimera.
o. As used herein, the term "binding partner" means those molecules that bind to one or more other molecules in a specific manner. Because the binding partner interactions are specific, there is a degree of selectivity that is achieved depending on the nature of the binding partners chosen. Non-limiting examples of binding partner complexes (formed from the component binding partners) include antzbody/antigen interactions, nucleic acid/nucleic acid interactions, enzyme/substrate interactions and receptor/ligand interactions. A non-limiting list of ligands includes avidin (and its analogs such as Streptavidin and Lumavidin^{™}), lectins, carbohydrates, peptides and proteins. The preferred pair of binding partners used in the practice of this invention is the antibody/antigen.
p. As used herein, the term "solid carrier" means an object that has a surface that is broad enough to accommodate organisms linked thereto. Preferred materials used to construct the solid carrier include, but are not limited to, glass, quartz, plastic (e.g. polystyrene, polyamide, polyacrylic, polyethylene, polypropylene and PTFE (Teflon)) and gold. The preferred solid carriers are particles, beads, microscope slides, micro titre plates, membranes and arrays.
q. As used herein, an "array" is a two or three-dimensional object having one or more surfaces upon which two or more unique, identifiable locations are created. A microscope slide is one example of an object that can be used to manufacture an array.

### II. General:

### Nucleic Acid Synthesis and Modification

Nucleic acid oligomer (oligonucleotide and oligoribonucleotide) synthesis has become routine. For a detailed-description of nucleic acid synthesis please see Gait, M. J., Oligonucleotide Synthesis: a Practical Approach. IRL Press, Oxford Engl*and.* Those of ordinary skill in the art will recognize that both labeled or unlabeled oligonucleotides (DNA, RNA and synthetic analogues thereof) are readily available. They can be synthesized using commercially available instrumentation and reagents or they-can be purchased from commercial vendors of custom manufactured oligonucleotides. Patents that discuss various compositions, supports and methodologies for the synthesis and labeling of nucleic acids include: 5,476,925, 5,453,496, 5,446,137, 5,419,966, 5,391,723, 5,391,667, 5,380,833, 5,348,868, 5,281,701, 5,278,302, 5,262,530, 5,243,038, 5,218,103, 5,204,456, 5,204,455, 5,198, 540, 5,175,209, 5,164,491, 5,112,962, 5,071,974, 5,047,524, 4,980,460, 4,923,901, 4,786,724, 4,725,677, 4,659,774, 4,500,707, 4,458,066, and 4,415,732.

### PNA Synthesis:

Methods for the chemical assembly of PNAs are well known (See: Patent Nos. 5,539,082, 5,527,675, 5,623,049, 5,714,331, 5,718,262, 5,736,336, 5,773,571, 5,766,855, 5,786,461, 5,837,459, 5,891,625, 5,972,610, 5,986,053 and 6,107,470 (Also see: PerSeptive Biosystems Product Literature)). Chemicals and instrumentation for the support bound automated chemical assembly of peptide nucleic acids are now commercially available. Both labeled and unlabeled PNA oligomers are likewise available from commercial vendors of custom PNA oligomers. Chemical assembly of a PNA is analogous to solid phase peptide synthesis, wherein at each cycle of assembly the oligomer possesses a reactive alkyl amino terminus that is condensed with the next synthon to be added to the growing polymer. Because standard peptide chemistry is utilized, natural and non-natural amino acids are routinely incorporated into a PNA oligomer. Because a PNA is a polyamide, it has a C-terminus (carboxyl terminus) and an N-terminus (amino terminus). For the purposes of the design of a hybridization probe suitable for antiparallel binding to the target sequence (the preferred orientation), the N-terminus of the probing nucleobase sequence of the PNA probe is the equivalent of the 5'-hydroxyl terminus of an equivalent DNA or RNA oligonucleotide.

### PNA Labelling:

Preferred non-limiting methods for labeling PNAs are described in US 6,110,676, WO99/22018, WO99/21881, WO99/49293 and WO99/37670, the examples section of this specification or are otherwise well known in the art of PNA synthesis and peptide synthesis.

### Labels:

Non-limiting examples of detectable moieties (labels) suitable for directly labeling molecular probes, antibodies or antibody fragments used in the practice of this invention include a dextran conjugate, a branched nucleic acid detection system, a chromophore, a fluorophore, a spin label, a radioisotope, an enzyme, a hapten, an acridinium ester and a chemiluminescent compound. Other suitable labeling reagents and preferred methods of attachment would be recognized by those-of ordinary skill in the art of PNA, peptide or nucleic acid synthesis.

Preferred haptens include 5(6)-carboxyfluorescein, 2,4-dinitrophenyl, digoxigenin, and biotin.

Preferred fluorochromes (fluorophores) include 5(6)-carboxyfluorescein (Flu), 6-((7-amino-4-methylcoumarin-3-acetyl)amino)hexanoic acid (Cou), 5(and 6)-carboxy-X-rhodamine (Rox), Cyanine 2 (Cy2) Dye, Cyanine 3 (Cy3) Dye, Cyanine 3.5 (Cy3.5) Dye, Cyanine 5 (Cy5) Dye, Cyanine 5.5 (Cy5.5) Dye Cyanine 7 (Cy7) Dye, Cyanine 9 (Cy9) Dye (Cyanine dyes 2, 3, 3.5, 5 and 5.5 are available as NHS esters from Amersham, Arlington Heights, IL), JOE, Tamara or the Alexa dye series (Molecular Probes, Eugene, OR).

Preferred enzymes include polymerases (e.g. Taq polymerase, Klenow PNA polymerase, T7 DNA polymerase, Sequenase, DNA polymerase 1 and phi29 polymerase), alkaline phosphatase (AP), horseradish peroxidase (HRP) and most preferably, soy bean peroxidase (SBP).

### Detectable and Independently Detectable Moieties/Multiplex Analysis:

In preferred embodiments of this invention, a multiplex hybridization assay is performed. In a multiplex assay, numerous conditions of interest are simultaneously or sequentially examined. Multiplex analysis relies on the ability to sort sample components or the data associated therewith, during or after the assay is completed. In preferred embodiments of the invention, one or more distinct independently detectable moieties are used to label two or more different molecular probes used in an assay. The ability to differentiate between and/or quantitate each of the independently detectable moieties provides the means to multiplex a hybridization assay because the data that correlates with the hybridization of each of the distinct, independently labeled molecular probe to a particular target sequence can be correlated with the presence, absence or amount of each organism sought to be detected in the sample. Consequently, the multiplex assays of this invention may be used to simultaneously or sequentially detect the presence, absence or quantity of two or more organisms in the same sample and in the same assay.

### Unlabeled Molecular Probes:

The PNA are used for the practice of this invention need not be labeled with a detectable moiety to be operable within the methods of this invention. It is possible to detect the PNA target sequence complex formed by hybridization of the PNA to the target sequence using an anybody raised to bind to the probe/target sequence complex. As a non-limiting example, a PNA/nucleic acid complex may be detected using an antibody that specifically interacts with the complex, under suitable antibody binding conditions. Suitable antibodies to PNA/nucleic acid complexes and methods for their preparation and use are described in WIPO Patent Application WO95/17430 as well as US 5,612,458. Antibodies to DNA/DNA hybrids are well known in the art and can be made and used as described in US 5,200,313.

### Self-Indicating "Beacon" Probes:

The labels attached to "Beacon" probes comprise a set (hereinafter "Beacon Set(s)") of energy transfer moieties having at least one energy transfer donor and at least one energy transfer acceptor moiety. Typically, the Beacon-Set will include a single donor moiety and a single acceptor moiety. Nevertheless, a Beacon Set may contain more than one donor moiety and/or more than one acceptor moiety. The donor and acceptor moieties operate such that one or more acceptor moieties accepts energy transferred from the one or more donor moieties or otherwise quenches the signal from the donor moiety or moieties. Though the previously listed fluorophores (with suitable spectral properties) might also operate as energy transfer acceptors, preferably, the acceptor moiety is a quencher moiety. Preferably, the quencher moiety is a non-fluorescent aromatic or heteroaromatic moiety. The preferred quencher moiety is 4-((-4-(dimeflylamino)phenyl)azo) benzoic acid (dabcyl).

Transfer of energy between donor and acceptor moieties of a "Beacon" probe may occur through collision of the closely associated moieties of a Beacon Set(s) or through a non radiative process such as fluorescence resonance energy transfer (FRET). For FRET to occur, transfer of energy between donor and acceptor moieties of a Beacon Set requires that the moieties be close in space and that the emission spectrum of a donor(s) have substantial overlap with the absorption spectrum of the acceptor(s) (See: Yaron et al. Analytical Biochemistry, 95:228-235 (1979) and particularly page 232, col. 1 through page 234, col. 1). Alternatively, collision mediated (radiationless) energy transfer may occur between very closely associated donor and acceptor moieties whether or not the emission spectrum of a donor moiety(ies) has a substantial overlap with the absorption spectrum of the acceptor moiety(ies) (See: Yaron et al., Analytical Biochemistry, 95: 228-235 (1979) and particularly page 229, col. 1 through page 232, col. 1). This process is referred to as intramolecular collision since it is believed that quenching is caused by the direct contact of the donor and acceptor moieties (See: Yaron et al.).

### (i) Linear Beacons:

In a preferred embodiment, the self-indicating "Beacon" probe is a Linear Beacon as more fully described in co-pending and commonly owned patent application USSN 09/179,162 (now allowed), entitled: "Methods, Kits And Compositions Pertaining To Linear Beacons", herein incorporated by reference.

### (ii) PNA Molecular Beacons:

In a preferred embodiment, the self-indicating "Beacon" probe is a PNA Molecular Beacon as more fully described in co-pending patent application: USSN 09/179,298, entitled:
"Methods, Kits And Compositions Pertaining To PNA Molecular Beacons", herein incorporated by reference.

### (iii) DNA Molecular Beacons:

In a preferred embodiment, the self-indicating "Beacon" probe is a nucleic acid molecular beacon as more fully described in US 5,925,517, entitled: "Detectably Labeled Dual Conformation Oligonucleotide Probes, Assays and Kits".

### Detecting Energy Transfer:

Hybrid formation of a self-indicating "Beacon" probe with a target sequence can be monitored by measuring at least one physical property of at least one member of the Beacon Set that is detectably different when the hybridization complex is formed as compared with when the "bacon" probe exists in the absence of target sequence. We refer to this phenomenon as the self-indicating property of "Beacon" probes. This change in detectable signal results from the change in efficiency of energy transfer between the donor and acceptor caused by hybridization of the "Beacon" probe to the target sequence. Preferably, the means of detection will involve measuring fluorescence of a donor or acceptor fluorophore of a Beacon Set. Most preferably, the Beacon Set will comprise at least one donor fluorophore and at least one acceptor quencher such that the fluorescence of the donor fluorophore is used to detect, identify or quantitate hybridization of the probe to the target sequence.

### Other Self-Indicating Probes:

In another embodiment, the self-indicating probes of this invention are of the type described in WIPO patent application WO97/45539. The self-indicating probes described in WO97/455 differ as compared with "Beacon" probes in that no quencher or acceptor is required because the reporter group must interact with the nucleic acid to thereby produce a detectable signal. Preferably, the probes of WO97/45539, as used in this invention, are appropriately labeled peptide nucleic acids that produce detectable signal upon hybridization to the target sequence.

### Spacer/Linker moieties:

Generally, spacers are used to minimize the adverse effects that bulky labeling reagents might have on hybridization properties of probes. Linkers typically induce flexibility and randomness into the probe or otherwise link two or more nucleobase sequences of a molecular probe. Preferred spacer/linker moieties for the nucleobase polymers of this invention consist of one or more aminoalkyl carboxylic acids (e.g. aminocaproic acid) the side chain of an amino acid (e.g. the side chain of lysine or ornithine) natural amino acids (e.g. glycine), aminooxyalkylacids (e.g. 8-amino-3,6-dioxaoctanoic acid), alkyl diacids (e.g. succinic acid), alkyloxy diacids (e.g. diglycolic acid) or alkyldiamines (e.g. 1,8-diamino-3,6-dioxaoctane). Spacer/linker moieties may also incidentally or intentionally be constructed to improve the water solubility of the molecular probe (For example see: Gildea et al., Tett. Lett. 39: 7255-7258 (1998)). Preferably, a spacer/linker moiety comprises one or more linked compounds having the formula: -Y-(Oₘ-(CW₂)ₙ)ₒ-Z-. The group Y is selected from the group consisting of: a single bond, -(CW₂)ₚ-,-C(O)(CW₂)ₚ-, -C(S)(CW₂)ₚ-and-S(O₂)(CW₂)ₚ. The group Z has the formula NH, NR², S or O. Each W is independently H, R², -OR², F, Cl, Br or I; wherein, each R² is independently selected from the group consisting of: -CX₃, -CX₂CX₃,-CX₂CX₂CX₃, -CX₂CX(CX₃)₂ and-C(CX₃)₃. Each X is independently H, F, Cl, Br or I. Each m is independently 0 or 1. Each n, o and p are independently integers from 0 to 10.

### Hybridization Conditions/Stringency:

Those of ordinary skill in the art of nucleic acid hybridization will recognize that factors-commonly-used to impose or control stringency of hybridization include formamide concentration (or other chemical denaturant reagent), salt concentration (i.e., ionic strength), hybridization temperature, detergent concentration, pH and the presence or absence of chaotropes. Optimal stringency for a molecular probe/target sequence combination is often found by the well-known technique of fixing several of the aforementioned stringency factors and then determining the effect of varying a single stringency factor. The same stringency factors can be modulated to thereby control the stringency of hybridization of a PNA to a nucleic acid, except that the hybridization of a PNA is fairly independent of ionic strength. Optimal stringency for an assay may be experimentally determined by examination of each stringency factor until the desired degree of discrimination is achieved.

### Suitable Hybridization Conditions

Generally, the more closely related the background causing nucleic acid contaminates are to the target sequence, the more carefully stringency must be controlled. Blocking probes may also be used as a means to improve discrimination beyond the limits possible by mere optimization of stringency factors. Suitable hybridization conditions will thus comprise conditions under which the desired degree of discrimination is achieved such that an assay generates an accurate (within the tolerance desired for the assay) and reproducible result. Aided by no more than routine experimentation and the disclosure provided herein, those of skill in the art will easily be able to determine suitable hybridization conditions for performing assays utilizing the methods and compositions described herein. Suitable *in-situ* hybridization conditions comprise conditions suitable for performing an *in-situ* hybridization procedure. Thus, suitable *in-situ* hybridization conditions will become apparent to those of skill in the art using the disclosure provided herein; with or without additional routine experimentation.

### Suitable Antibody Binding Conditions:

Suitable antibody binding conditions comprise conditions suitable for binding an antibody to its antigen. Thus, suitable antibody binding conditions will become apparent to those of skill in the art using the disclosure provided herein; with or without additional routine experimentation. By way of general guidance to the practitioner in determining suitable antibody binding conditions, methods for preparing and using antibodies can be found in numerous references including: Molecular Probes Of The Nervous System, Volume 1, "Selected Methods For Antibody and Nucleic Acid Probes", Cold Spring Harbor Laboratory Press, 1993 by S. Hockfield et al.

### Harmonization Of Suitable Hybridization Conditions & Suitable Antibody Binding

### Conditions:

When employing the methods of this invention or in the production of the compositions of this invention, it maybe important to harmonize the hybridization conditions with the antibody binding conditions because the staining of the organisms is performed simultaneously with, or subsequent to, an antibody binding event Because optimization of the same variables (pH; salt concentration etc.) is involved, aided by no more than routine experimentation, those of skill in the art will easily be able to harmonize the antibody binding conditions and suitable hybridization conditions for performing an assay. It should however be noted that PNA probes are used, providing harmonization of the hybridization and antibody binding conditions because PNA probe bind more tightly under conditions of physiological salt, conditions under which antibodies are more likely to operate most efficiently.

### Blocking Probes:

Blocking probes are nucleic acid or non-nucleic acid probes that can be used to suppress the binding of the probing nucleobase sequence of the molecular probe to a non-target sequence. Preferred blocking probes are PNA probes (See: Coull et al., US 6,110,676). Typically, blocking probes are closely related to the probing nucleobase sequence and preferably they comprise one or more single point mutations as compared with the detectable molecular probe that is complementary, or substantially complementary, to the target sequence sought to be detected in the assay. It is believed that blocking probes operate by hybridization to the non-target sequence to thereby form a more thermodynamically stable complex than is formed by hybridization between the probing nucleobase sequence and the non-target sequence. Formation of the more stable and preferred complex blocks formation of the less stable non-preferred complex between the probing nucleobase sequence and the non-target sequence. Thus, blocking probes can be used with the methods and compositions of this invention to suppress the binding of the molecular probe to a non-target sequence that might be present in the organism to be distinguished or otherwise interfere with the performance of the assay. Hence, blocking probes can be used to improve the fidelity of the methods of this invention beyond the limits available using the combined selectivity of binding partners and molecular probes. Blocking probes are particularly advantageous in single point mutation analysis (e.g. single nucleotide polymorphism (SNP) analysis).

### Probing Nucleobase Sequence:

The probing nucleobase sequence of a molecular probe is the specific sequence recognition portion of the construct. Therefore, the probing nucleobase sequence is a nucleobase sequence designed to hybridize to a specific target sequence wherein the presence, absence or amount of the target sequence can be used to directly or indirectly detect the presence, absence or number of organisms of interest in a sample. Consequently, with due consideration to the requirements of a molecular probe for the assay format chosen, the length and sequence composition of the probing nucleobase sequence of the probe will generally be chosen such that a stable complex is formed with the target sequence under suitable hybridization conditions.

### Probe Complexes:

Compositions comprising a PNA probe triplex have been described in copending and commonly owned application USSN 09/302,238 (also published as WO99/55916). Using this methodology, the probes that hybridize to the target sequence may or may not be labeled since it is the probe complex formed by the annealing of the adjacent probes that is directly detected and not the probes that directly bind to the target sequence.

### Organism Of Interest:

The assay user or designer will select the organism of interest. As used herein, the organism of interest is a microorganism, tissue or microscopic sized cell. The organism of interest may be a cell, bacteria, virus, yeast, fungi, other unicellular organism or a multicellular organism. Thus, there are no limitations in the organism of interest except that it be microscopic in size.

The organism of interest is generally selected to be an organism characterized by domain, kingdom, group, class, genus, species, taxon, subclass, subspecies, serotype, strain or by any other recognized means of characterization of the organism of interest. Optionally, but not necessarily, the organism of interest will be chosen such that it is to be distinguished from a closely related organism or organisms wherein an antibody or probe based assay, alone, is not adequate to properly characterize the organism of interest from the organism or organisms to be distinguished.

### Determining Particles Or Beads:

In certain embodiments of this invention, particles or beads are used as a solid carrier in the assay methods wherein organisms bound to the particles or beads are determined. In certain of these embodiments, it is further required that a property inherent to the particle composition be independently determined. For these assays, coded beaded supports are used.

### Coded Beaded Supports:

Coded beaded supports are particles or beads that possess an inherent independently detectable property that can be determined independently of the properties of constituents that are bound to the particle. Hence, many different types of coded beaded supports can be made independently detectable so that an assay can be designed to assign a parameter of interest to each different particle or bead type and thereby provide a way to determine that parameter by determining the particle type. Non-limiting examples of parameters that may be associated with (i.e. used to "code") the particle type include the sample source or another independent-assay parameter.

It is not intended that the coded beaded supports must be determined by instrument where the coded beaded supports can be determined without the aid of an instrument. For example, four coded beaded supports might comprise a visible red, blue, green and yellow color. Because these coded supports are visibly colored, they can be determined by visual analysis, or if desired, by instrument analysis.

### Particle Sorters And Sorting Particles:

In certain embodiments of this invention, it is required that particles or beads be sorted and that constituents thereon be determined. Any flow cytometer is a particle sorter suitable for determining characteristics of the constituents of micro particles or micro beads provided that the assay need not determine a coded beaded support. If the assay must determine a coded beaded support then the flow cytometer must be so equipped to make that determination unless the determination can be made visually or in another way.

As discussed in the Background section above, the Luminex 100^{™} represents an instrument capable of both determining the coded beaded support as well as determining properties of constituents bound to said particle. Thus, the instrument provides a means to multiplex assays such that each different type of coded beaded support can be used to code for a parameter (property) of interest or sample source and wherein the constituent or constituents bound to the particles or beads are analyzed simultaneously or sequentially for an independent property or properties. In the context of the present invention, the constituents bound to the particles are organisms or cells. It is noted that Luminex does not appear to teach or suggest that their coded beaded supports and/or instruments are suitable for the direct analysis of organisms or cells immobilized to said coded beaded supports.

### Microscope Slides:

In other preferred embodiments of this invention, the solid carrier is chosen to be a microscope slide. The microscope slide may be made of glass, quartz, plastic or other material that is translucent to light. Microscope slides are a useful solid carrier because the surface can be easily modified with a binding partner and then stained organisms, which are immobilized to the solid carrier, can be viewed in an appropriately equipped microscope. In certain preferred embodiments, a microscope slide can be manufactured to be an array by the application of the same or different binding partners at each of the unique, identifiable locations.

### Arrays:

In other preferred embodiments of this invention, the solid carrier is chosen to be an array. Arrays comprise unique, identifiable locations that differ in a way that can be used to determine a characteristic or property of interest. In the context of the present invention, the same or a different binding partner will be linked to each of the unique, identifiable locations of the array. In a preferred embodiment, the different binding partners are chosen to link a different organism of interest to each of the unique; identifiable locations of the array.

### Immobilizing Binding Partners To The Solid Carrier:

Numerous surface chemistries exist for the modification of surfaces for the purpose of producing surface bound functional groups to which binding partners can be linked for immobilization. Additionally, numerous solid carriers possessing reactive functional groups are commercially available. These include the coded beaded supports available from Luminex (See Luminex Product Literature), chromatographic packing materials that are available from vendors such as Amersham Pharmacia (See Amersham Pharmacia Catalog) and coated glass slides that are available from Corning and can be used to fashion micro arrays. (See: Corning MicroArray technology at www.corning,ny.com), Once the solid carrier is derivatized to possess available functional groups, the binding partner is easily reacted with the surface bound functional group to thereby effect binding partner immobilization. Those of ordinary skill in the art will further appreciate that any solid carrier can be derivatized using available surface chemistry technology to fashion a custom solid carrier bearing the desired immobilized binding partner or partners. Hence, this invention is not intended to be limited by any particular commercially available solid carrier or any particular surface chemistry modification.

### Advantages Of The Present Invention:

It is an advantage of the present invention that the selectivity and discriminating power of binding partners, as used in a capture assay, is combined with the selectivity and discriminating power of molecular probes, as used to stain organisms, tissues or cells, in a way that provides for two independent levels of certainty and/or discrimination in the assay. Hence, the assays and compositions of this invention provide for either or both a greater ability to discriminate organisms, tissues and/or cells and/or a greater deal of certainty with respect to the result of the assay.

### III Preferred Embodiments of the Invention:

### a. Assay Methods:

### (i) Method For Determining An Organism

In one embodiment, this invention is directed to a method for determining an organism of interest in a sample from another organism or organisms to be distinguished. The method comprises treating the sample, or a portion thereof, with at least one detectable molecular probe wherein the molecular probe or probes are peptide nucleic acid and are selected such that either: (i) both the organism of interest and the other organism or organisms react with the molecular probe in a way that produces detectable organisms of interest and a detectable other organism or organisms to be distinguished; or (ii) only the organism of interest reacts with the molecular probe in a way that produces only detectable organisms of interest. According to the method, the sample, or a portion thereof, is also contacted with a solid carrier to which has been immobilized a binding partner which is an antibody such that if (i) applies then the binding partner is chosen to be reactive only with the detectable organism of interest but not reactive with the detectable other organism or organisms to be distinguished; but if (ii) applies then the binding partner is chosen to be generally reactive with the detectable organism of interest but also may be reactive with the other organism or organisms to be distinguished. Once the organisms have been stained and immobilized to the solid carrier, the method involves determining the presence, absence, position or number of detectable organisms immobilized to the solid carrier and correlating the result with the presence, absence, or number of the organisms of interest in the sample, or portion thereof.

In determining the organism of interest, the method requires that a correlation between the presence, absence, position or number of detectable organisms immobilized to the solid carrier be made with the presence, absence or number of organisms of interest in the sample, or portion thereof. This correlation is straight forward since the organisms that are determined by operation of the method come directly from the sample, or portion thereof.

In preferred embodiments of this method, the molecular probe stains all organisms of a domain, kingdom, group, class, genus, species, taxon, subclass, subspecies, serotype or strain without regard to whether or not this represents the organism of interest. For this preferred embodiment, it is the binding partner that is specific for the domain, kingdom, group, class, genus, species, taxon, subclass, subspecies, serotype or strain that is the organism of interest.

As a non-limiting example of this embodiment, the molecular probe is selected to be suitable for staining all bacteria in the sample wherein the organism of interest is Salmonella bacteria. Thus, the use of a solid carrier immobilized binding partner that is an antibody to Salmonella bacteria will facilitate, for determination, the selective immobilization of only the stained Salmonella bacteria. Since the other organisms are not immobilized to the solid carrier, they are not determined by the method even if they are detectably stained. Hence, it is the interaction with the solid carrier that provides the ultimate selectivity for the organism of interest in this embodiment.

In another preferred embodiment of this method, the molecular probe stains only the domain, kingdom, group, class, genus, species, taxon, subclass, subspecies, serotype or strain that is the organism of interest. For this preferred embodiment, the binding partner is chosen to bind a particular domain, kingdom, group, class, genus, species, taxon, subclass, subspecies, serotype or strain without regard to whether or not this represents the organism of interest, provided however that the organism of interest is within the scope of the binding capability of the binding partner.

As a non-limiting example of this embodiment, the molecular probe is selected to be specific for the staining of Salmonella bacteria wherein Salmonella bacteria represents the organism of interest. Thus, the use of a solid carrier immobilized binding partner that is an antibody to all bacteria will facilitate, for determination, the selective immobilization of all bacteria. Although other bacteria may be captured by the solid carrier, they are not detectably labeled and are thus, not determined by the method. Hence, it is the presence of the stain that provides the ultimate selectivity for the organism of interest in this embodiment.

It should be noted that certainty at two levels of molecular discrimination is very useful since although binding partners and molecular probes are designed to be selective, they cannot be tested for cross reaction against all organisms or other interfering matter. Hence, even if some level of cross reactivity occurs in the assay with either of the molecular probe or binding partner, because the selectivity substantially differs at each different level of molecular discrimination, it is not likely that a particular cross reacting species will exhibit cross reactivity at both levels of discrimination. Consequently, the certainty of a result is significantly increased where a positive result requires, as does certain embodiments of this invention, that the assay perform an interrogation at two levels of molecular discrimination.

### (ii) Method For Sorting And Determining An Organism Using Coded Beaded Supports

In another embodiment, this invention is directed to a method for sorting and determining an organism or organisms of interest in a sample or samples using coded beaded supports. The method is as defined in the claims and comprises treating the sample, or a portion thereof, with one or more detectable or independently detectable molecular probes wherein the one or more molecular probes are selected such that either. (i) the detectable probe or probes react with the different organisms to be determined in a way that produces different detectable organisms that possess the same stain; or (ii) the independently detectable probes react with the different organisms to be determined in a way that produces different independently detectable organisms that possess an independently detectable stain. According to the method, the sample, or a portion thereof, is also contacted with one or more different types of coded beaded supports, wherein each different type of coded beaded support can be independently determined in a suitable particle sorter and wherein to each different type of coded beaded support has been immobilized a particular binding partner that is chosen to select a particular organism or organisms such that detectable or independently detectable organisms become selectively bound to me coded beaded supports as a result of the occurrence of specific binding partner interactions. According to the method, the different types of coded beaded supports are then sorted in a suitable particle sorter. The presence, absence, or number of the detectable organism or organisms, or each of the independently detectable organisms, immobilized to each different type of coded beaded support is also determined. This result is then either: (iii) correlated with the code that is associated with a particular immobilized binding partner to thereby determine the presence, absence or number of each of the different organisms of interest in the sample or portion thereof; or (iv) correlated with the code for a sample source from which the sample, or portion thereof, was derived to thereby determine the presence, absence or number of each of the different organisms of interest in each different sample, or portion thereof.

In determining the organism or organisms of interest, the present method requires that a correlation between the presence, absence, or number of detectable organisms immobilized to the coded beaded support be made with the presence, absence or number of organisms of interest in the sample, or portion thereof. This correlation is straight forward since the organisms that are determined by operation of the method come directly from the sample, or portion thereof.

However, certain embodiments of the present method also require that a correlation be made between the "code" of the coded beaded support and a particular binding partner. Since the coded beaded supports are manufactured, the identity and properties of the binding partner immobilized thereto is predetermined and known. Consequently, this correlation is again straight forward.

Additionally, certain other embodiments of the present method require that a correlation be made between the "code" of the beaded support and the source of a particular sample, or portion thereof. Because for this embodiment each sample is assigned to a particular coded support and because the "code" for each sample is predetermined and known, the resulting correlation is also straight forward.

In one preferred embodiment of this method, the detectable molecular probe or probes stain the organism or organisms of interest with the same stain. For this embodiment, the binding partner that is associated with each different type of coded beaded support is chosen to be specific for one of the different organisms of interest such that the sorting of the different types of coded beaded supports determines each of the different organisms of interest in a sample, or portion thereof, based upon the identity of each different binding partner that is associated with each particular coded beaded support.

As a non-limiting example of this embodiment, the molecular probe is chosen to stain all bacteria in the sample, or portion thereof, wherein the organism of interest for one of the coded beaded supports is Salmonella. Thus, the use of a coded beaded support (Bead Type 1) to which is linked an antibody that is specific to Salmonella bacteria, will facilitate the selective immobilization of only the stained Salmonella bacteria to Bead Type 1. Since this coded beaded support is associated only with the presence of the Salmonella antibody, a determination of the code of the bead (Bead Type 1), in combination with a determination of stained organisms on said bead, provides all the information needed to make a determination of presence, absence or number of Salmonella bacteria in the sample.

It should be noted that the same sample can also be analyzed for other bacteria such as, for example, Pseudomonas aeruginosa. According to the method, a second coded beaded support (Bead Type 2), to which is linked an antibody that is specific to Pseudomonas aeruginosa bacteria, will facilitate the selective immobilization of only the stained Pseudomonas aeruginosa bacteria to Bead Type 2. Since all the bacteria of the sample, or portion thereof, are stained with the same color, a determination of the "code" of the bead (Bead Type 2) in combination with a determination of stained organisms on the bead, provides all the information needed to make a determination of the presence, absence or number of Pseudomonas aeruginosa bacteria in the sample. Hence, the method provides for multiplex analysis since many different coded beaded supports can be used for the analysis of numerous different organisms of interest that might be present in the same sample.

Moreover, it will also become apparent to the ordinary practitioner that the number of different organisms of interest that can be detected from the same sample using this methodology is limited only by the availability of different bead types and the availability of specific binding partner complexes. Nevertheless, even this is not a true limitation since antibodies can be raised to most antigens and coded beads can be custom made.

In another preferred embodiment of this method, the independently detectable molecular probe or probes stain all of the organisms of interest provided that some or all of the different organisms of interest are stained differently. For this embodiment, each binding partner associated with each different type of coded beaded support is chosen to select among the same or differently stained organisms such that the sorting of the different types of coded beaded supports, when considered in combination with the stain of the organism or organisms bound to each different type of coded beaded support, is used to determine each of the different organisms of interest in a sample, or portion thereof.

As a non-limiting example of this embodiment, each of the independently detectable molecular probes are selected to be specific for staining different organisms of interest such that the different organisms of interest are independently detectable when stained. For example, the different organisms of interest are Salmonella bacteria, E. coli bacteria and Pseudomonas aeruginosa bacteria wherein the bacteria are stained, using suitable fluorophore labeled molecular probes, with red, green and blue fluorophores, respectively. For this example, the binding partner is selected to be: (a) suitable for binding the bacteria to the same coded beaded support; or (b) suitable for binding individual bacterial strains to the same or different coded beaded supports. As evident by the example described below, the assay can be utilized whereby both (a) and (b) apply. Thus, it is not clearly not a limitation of this embodiment of the invention that either (a) or (b) apply.

For simplicity, assume that the binding partner on Bead 1 is specific for E. coli and the binding partner on Bead 2 is specific for both Salmonella bacteria and Pseudomonas aeruginosa bacteria. In this way both conditions (a) and (b) are generally met. Since the color of the organisms bound to the solid carrier provides one level of selectivity, and the nature of the binding partner associated with different coded beaded supports provides a second level of selectivity, a determination of both the color or colors of organisms immobilized to the beads and the "code" of each bead upon which the color determination has been made, provides all the information necessary to determine the presence, absence or amount of the different organisms in the sample, or portion thereof.

More specifically, if situation (a) applies the presence, absence or quantity of red, green or blue fluorophore detected on the only type of coded beaded support determines the presence, absence or number of Salmonella bacteria, E. coli bacteria and Pseudomonas aeruginosa bacteria in the sample, or portion thereof. Consequently, for this embodiment, the ultimate selectivity is achieved using the molecular probe. However, if situation (b) applies, then the beads can be interrogated for extraneous colors. In the present example, Bead 1 need only be analyzed for green (E. coli), and Bead 2 need only be analyzed for red (Salmonella bacteria) and blue (Pseudomonas aeruginosa bacteria). Hence, in this embodiment of a multiplex assay, selectivity is achieved at both the level of interaction of the molecular probe and again at the level of the binding partner as determined by the nature of each different coded beaded support. Therefore, the certainty of the result is improved in situation (b).

In yet another preferred embodiment of this method, the independently detectable molecular probes stain all of the organisms of interest differently. For this embodiment, the binding partner associated with each different type of coded beaded support is the same. However, each different coded beaded support "codes" for a different sample such that the determination of the stain or stains on each different coded beaded support specifically determines each of the different organisms of interest in the sample, or portion thereof, and each different coded beaded support identifies the source of the sample, or portion thereof. Hence, the determination of the color of organisms on each coded beaded support, when considered with the bead "code", provides all the information needed to determine the organisms in the sample and the sample source, respectively.

As a non-limiting example of this embodiment, 100 different coded beaded supports are all derivatized with an antibody or antibodies that is/are suitable for the capture of 5 different organisms of interest. To each sample to be tested is added five different fluorophore labeled molecular probes under conditions that will, if present, stain each of the five different bacteria of interest one of either, blue, yellow, orange, red or green. To each of 100 different samples that are to be tested is added one of the 100 different coded beaded supports such that all 100 samples are identifiable by a different one of the 100 different coded beaded supports. Since each coded beaded support now "codes" for a different sample, the coded beaded supports can be analyzed simultaneously (in a mixture) or sequentially to thereby obtain the result for the many different samples, or portions thereof. Consequently, this embodiment of the method pertains to multiplexing samples and optionally multiplexing the analysis of sample analytes; it being noted that the sample analytes (each of the five different organisms of interest) are multiplexed by using the five different independently detectable molecular probes and each different sample source being multiplexed using independently determinable coded beaded supports. Hence, in this example, multiple levels of multiplexing of the assay is performed such that throughput of the system is substantially improved.

It should be noted, that the number of different organisms of interest that can be determined is limited only by the number of different independently detectable molecular probes that can be prepared and that five organisms per sample is not intended to be a limit on the method. Furthermore, it is evident that 100 different types of coded beaded supports is not intended to be a limit on the method.

### (iii) Method For Sorting And Determining An Organism Using An Array

In yet another embodiment, this invention is directed to a method for sorting and determining different organisms of interest in a sample using an array. The method is as defined in the claims and comprises treating the sample, or a portion thereof, with one or more detectable or independently detectable molecular probes wherein the one or more molecular probes are selected such that either: (i) the detectable probe or probes react with the different organisms to be determined in a way that produces different detectable organisms that possess the same stain; or (ii) the independently detectable probes react with the different organisms to be determined in a way that produces different independently detectable organisms that possess an independently detectable stain. According to the method, the sample, or a portion thereof, is contacted with a solid carrier array to which binding partners have been immobilized at unique, identifiable locations such that the detectable or independently detectable organisms are selectively bound to the locations on the array as a result of the occurrence of specific binding partner interactions. The presence, absence or number of the detectable or independently detectable organisms immobilized at the many different locations of the array is then determined and the result is correlated with the particular binding partner immobilized to each location on the array to thereby determine the presence, absence or number of the different organisms of interest in the sample.

In determining the organism of interest, the method requires that a correlation between the presence, absence, position or number of detectable organisms immobilized to the solid carrier be made with the presence, absence or number of organisms of interest in the sample, or portion thereof. This correlation is straight forward since, although the array provides for sorting, the organisms that are determined by operation of the method come directly from the sample, or portion thereof.

In one embodiment of this method, the detectable molecular probe or probes stain all of the different organisms with the same stain. For this embodiment, the binding partner is chosen to be specific for each of the different organisms of interest such that the sorting of the organisms on the array moulting from the binding partner interactions occurring at the unique locations is used to thereby determine each of the different organisms of interest in the sample, or portion thereof, based solely upon the identity of the different binding partners at the unique locations. Because the array is fabricated, the identity of each binding partner at a unique, identifiable location is predetermined and known.

As a non-limiting example of this embodiment, a molecular probe is chosen to stain all bacteria in the sample with a red fluorophore. Additionally, an array of three different binding partners is prepared such that at each unique, identifiable location (a "spot") on the array, a different one of three binding partners, to each of Salmonella bacteria, E. coli bacteria and Pseudomonas aeruginosa bacteria, is immobilized. Thus, the array can be said to comprise a Salmonella spot, an E. coli spot and a Pseudomonas aeruginosa spot. Consequently, a determination of the red fluorophore at one or more of the "spots" on the array, when correlated with the identity of the binding partner immobilized at the unique identifiable location, provides the information necessary to determine the presence, absence or amount of each of Salmonella bacteria, E. coli bacteria and Pseudomonas aeruginosa bacteria in the sample, or portion thereof.

In still another embodiment of this method, the independently detectable molecular probe or probes stain all of the organisms of interest provided that some or all of the different organisms of interest are stained differently. For this embodiment, each binding partner associated with a unique location on the array is chosen to select among the same or differently stained organisms such that the sorting of the organisms on the array resulting from the binding partner interactions occurring at the unique locations, when considered in combination with the stain of the organism or organisms bound to each unique location, is used to determine each of the different organisms of interest in the sample, or portion thereof.

As a non-limiting example of this embodiment, two or more independently detectable molecular probes are chosen to stain Salmonella bacteria, E. coli bacteria and Pseudomonas aeruginosa bacteria in the sample, or portion thereof with a red, green or blue fluorophore, respectively. Additionally, an array of two different binding partners is prepared such that at each of two unique, identifiable locations (a "spots") on the array, Salmonella bacteria and E. coli bacteria bind to the binding partner at spot 1 and Pseudomonas aeruginosa bacteria binds to the binding partner at spot 2. Thus, a determination of a red, green or blue fluorophore at each "spot" on the array, when correlated with the identity of the binding partner immobilized at the unique, identifiable location, provides the information necessary to determine the presence, absence or amount of each of Salmonella bacteria, E. coli bacteria and Pseudomonas aeruginosa bacteria in the sample, or portion thereof. In particular, a red (Salmonella) and/or green (E. coli) signal should be determined at spot 1 and a blue (Pseudomonas aeruginosa) signal should be determined at spot 2. It should be noted that in this embodiment of the method, the certainty of the assay is increased because the selectivity for some of the organisms is achieved at both the level of the molecular probe and at the level of the chosen binding partner. Hence, this is yet another example of the flexibility in Multiplexing that is available with the presently described inventive methods.

### (iv) General Attributes Of The Aforementioned Methods: .

According to the methods, the detectable molecular probe is a peptide nucleic acid oligomer.

According to the method, the detectable molecular probe need not labeled with a detectable moiety. For these embodiments, the detectable molecular probe may be detected using a detectable antibody that specifically binds to a detectable molecular probe/target sequence complex. For these embodiments, the detectable molecular probe is an unlabeled peptide nucleic acid and the peptide nucleic acid/target sequence is detected with a suitable detectable antibody.

In other preferred embodiments of this invention, the detectable molecular probe is labeled with a detectable moiety. The preferred detectable moiety suitable for the practice of this invention include: a chromophore, a fluorochrome, a spin label, a radioisotope, an enzyme, a hapten and a chemiluminescent compound.

It is generally not important to the success of any of the aforementioned methods whether or not the staining with molecular probes is performed before, during or after the immobilization of the particle, cell or tissue to the solid carrier provided however that both steps are performed before the determination of organism or organisms is made. Thus, in one embodiment the sample, or portion thereof, is treated with the detectable molecular probe or probes before being contacted with the solid carrier. In another embodiment, the sample, or portion thereof, is contacted with the solid carrier before being treated with the detectable molecular probe or probes. In yet another embodiment, the sample, or portion thereof, is simultaneously contacted with both the solid carrier and treated with the detectable molecular probe or probes.

### b. Compositions:

In still another embodiment, this invention is directed to a composition comprising one or more organisms stained with one or more detectable molecular probes wherein the molecular probe or probes are peptide nucleic acid and a solid carrier to which is immobilized a binding partner which is an antibody, wherein the one or more organisms are linked to the solid carrier through the interaction of the organism and its binding partner. In other preferred embodiments, the solid carrier is a solid carrier array comprising antibodies to many different organisms of interest which have each been immobilized at unique identifiable locations on the array. Alternatively, the solid carrier is a coded beaded support, a microscope slide or a membrane.

In still another embodiment, this invention is directed to a composition as defined in the claims comprising two or more different organisms of interest that are detectably or independently stained with one or more molecular probes and a mixture of two or more different types of coded beaded supports. To each different type of coded beaded support has been immobilized a different binding partner that is selected to detect a particular organism of interest and wherein the different detectable or independently detectable organisms are selectively bound to the coded beaded supports as a result of the occurrence of specific binding interactions of the binding partner and the organisms.

In yet another embodiment, this invention is directed to a composition comprising two or more different organisms of interest that are detectably or independently stained with one or more molecular probes and a solid carrier array to which binding partners have been immobilized at unique identifiable locations such that the detectably or independently stained organisms are selectively bound to the locations on the array as a result of the occurrence of specific binding partner interactions.

Having described the preferred embodiments of the invention, it will now become apparent to one of skill in the art that other embodiments incorporating the concepts described herein may be used. It is felt, therefore, that these embodiments should not be limited to disclosed embodiments and examples but rather should be limited only by the scope of the following claims.

### Brief Description of the Drawings

Figure 1A is a print of a composite digital image of the red and green images taken with a microscope equipped with a CCD camera. The red spheres in the image are coded beaded supports and the green rods are Salmonella choleraesuis bacteria immobilized to the surface of the beads.
Figure 1B is a print of a composite digital image of the red and green images taken with a microscope equipped with a CCD camera. The red sphere in the image is a coded beaded support and the green rods are Listeria monocytogenes bacteria that do not appear to bind to the surface of the bead.
Figure 2A is a print of a composite digital image of the red and green images taken with a microscope equipped with a CCD camera. The red sphere in the image is a coded beaded support and the green rods are Salmonella choleraesuis bacteria immobilized to the surface of the beads.
Figure 2B is a print of a composite digital image of the red and green images taken with a microscope equipped with a CCD camera. The red spheres in the image are coded beaded supports and the green rods are Listeria monocytogenes bacteria immobilized to the surface of the beads.

### Modes For Carrying Out The Invention

This invention is now illustrated by the following examples that are not intended to be limiting in any way.

### Example 1:

### PNA Oligomers As Molecular Probes:

PNA Oligomers where prepared from commercial reagents and instrumentation obtained from Applied Biosystems, Foster City, CA using well known methods.

### PNA Oligomers Prepared:

| **Table 1** | | |
|---|---|---|
| **Probe ID** | **PNA Probe Sequence** | **Seq. Id. No.** |
| Bac Uni | Flu-OO-CTG-CCT-CCC-GTA-GGA-NH₂ | 1 |

| | | |
|---|---|---|
| All PNA sequences are written from the amine (N-) terminus to the carboxyl (C-) terminus. Flu = 5(6)-carboxyfluorescein; O = 8-amino-3,6-dioxaoctanoic acid | | |

### Experimental Methods:

### Fixation of Cells

*Salmonella choleraesuis* and *Listeria monocytogenes* cells were fixed by pelleting 10-20 mL of exponentially growing cultures by centrifugation at 10,000 rpm for 5 minutes. The media was removed and cell pellets were resuspended in an equal volume of Buffer A. Cell suspensions were centrifuged at 10,000 rpm for 5 minutes, the supernatant was removed. Cells were resuspended in an equal volume of Buffer B, incubated at room temperature for 1 hour, pelleted by centrifugation at 10,000 rpm and washed with Buffer A. Final cell pellets were resuspended in an equal volume of Buffer C and stored at -20°C for a minimum of 30 minutes.

### Buffer Solutions

| | |
|---|---|
| A | 130 mM NaCl, 7 mM Na₂HP0₄, 7 mM NaH₂PO₄, pH 7.0 |
| B | 4% paraformaldehyde in Buffer A |
| C | 50% ethanol in water |
| D | 25 mM Tris pH 9.0,0.5% SDS, 100mM NaCl |
| E | 10 mM Tris pH 9.0,1 mM EDTA |

### Hybridization

For hybridization, 100 µL of fixed cells were pelleted by centrifugation for 5 minutes at 10,000 rpm, resuspended in 100 µl of Buffer A, pelleted as described above and finally resuspended in 100 µL of Buffer D. Fluorescein labeled Bac Uni PNA probe was then added to a final concentration of 300 pmole/mL. Reactions were then incubated at 55°C for 30 minutes. Reactions were pelleted as described above and pellets were washed for 10 minutes at 55°C in 500 µL Buffer E, followed by centrifugation at 10,000 rpm for 5 minutes. The wash step was repeated twice for a total of 3 washes. Final cell pellets were resuspended in 100 µL of Buffer E.

### Capture Onto Antibody Coated Beads

Two types of coded beads were received from Luminex, one coated with Salmonella-specific antibody (OEM Concepts, Toms River, NJ; the "Salmonella beads") and one with Listeria-specific antibody (OEM Concepts, Toms River, NJ; the "Listeria beads"). A 25 µL aliquot of each type of coded bead was pelleted by centrifugation and resuspended in 25 µL of Buffer E. The resuspended beads and the hybridization reactions were combined in equal volumes (5 µl of each) and incubated at room temperature with shaking for 2 hours. Capture reactions included treatment with S. *choleraesuis* for each coded bead type and treatment with *L. monocytogenes* for each bead type. Reactions were analyzed by spreading 2 µL of the sample on a preheated microscope slide (∼60°C). The slide was then dried on a heat block set at approximately 60°C. A 2 µL aliquot of mounting media (Vector Laboratories) was then added and a coverslip applied. All slides were then examined microscopically using a 60X objective and a double band pass filter (FITC/Texas Red).

### Results:

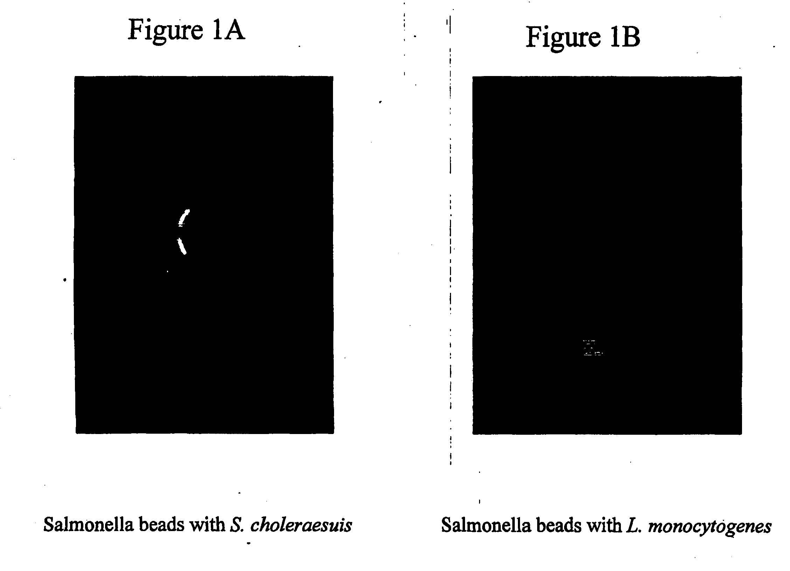

With reference to Figure 1, the Salmonella beads (red spheres) effectively captured the green stained *S*. *choleraesuis* cells (Figure 1A) and did not bind to the green stained L. *monocytogenes* cells (Figure 1B). By microscopic examination, it appears that approximately 25% of the beads were bound to S. *choleraesuis* cells, compared to <1% of beads bound to L. *monocytogenes.* With reference to Figure 2, while the Listeria beads (red spheres) did capture the green stained *L. monocytogenes* cells (Figure 2A), the *S. choleraesuis* cells are also captured by the Listeria specific beads (Figure 2B). It is possible that the non-specific binding seen with the Listeria beads occurs because the capture conditions were not optimized. Alternatively, the lack of specificity with the Listeria beads could be due a lack of specificity of the antibody used to coat the beads. This result therefore reinforces why selectivity at multiple levels of molecular discrimination is a preferred means of analysis. Future experimentation is planed to determine why the selected specificity was not achieved with the Listeria beads.

## Claims

1. A method for determining an organism of interest in a sample from another organism or organisms to be distinguished; said method comprising;
treating the sample, or a portion thereof, with at least one detectable molecular probe wherein the molecular probe or probes are peptide nucleic acid and are selected such that either:
(i) both the organism of interest and the other organisms react with the molecular probe in a way that produces detectable organisms of interest and a detectable other organism or organisms to be distinguished; or
(ii) only the organism of interest reacts with the molecular probe in a way that produces only detectable organisms of interest; and
contacting the sample, or a portion thereof, with a solid carrier to which has been immobilised a binding partner which is an antibody such that if (i) applies then the binding partner is chosen to be reactive only with the detectable organism of interest but not reactive with the detectable other organism or organisms to be distinguished; but if (ii) applies then the binding partner is chosen to be generally reactive with the detectable organism of interest but also may be reactive with the other organism or organisms to be distinguished; and
determining the presence, absence, position or number of detectable organisms immobilised to the solid carrier and correlating the result with the presence, absence, or number of the organisms of interest in the sample, or portion thereof.

2. The method of Claim 1, wherein the detectable molecular probe is not labelled with a detectable moiety.

3. The method of Claim 2, wherein the detectable molecular probe is detected using a detectable antibody that specifically binds to a detectable molecular probe/target sequence complex.

4. The method of Claim 1, wherein the detectable molecular probe is labelled with a detectable moiety.

5. The method of Claim 4, wherein the detectable moiety is selected from the group consisting of: a chromophore, a fluorochrome, a spin label, a radioisotope, an enzyme, a hapten and chemiluminescent compound.

6. The method of Claim 1, wherein the solid carrier is selected from the group consisting of: a particle, a bead, a microscope slide, a micro titre plate, a membrane and an array.

7. The method of Claim 1, wherein the sample, or portion thereof, is treated with the detectable molecular probe or probes before being contacted with the solid carrier.

8. The method of Claim 1, wherein the sample, or portion thereof, is contacted with the solid carrier before being treated with the detectable molecular probe or probes.

9. The method of Claim 1, wherein the sample or portion thereof, is simultaneously contacted with both the solid carrier and treated with the detectable molecular probe or probes.

10. A method as claimed in any one of Claims 1 to 5 or 7 to 9, wherein the solid carrier comprises two or more different types of coded beaded supports, wherein each different type of coded beaded support can be independently determined in a suitable particle sorter, wherein to the coded beaded supports have been immobilised one or more binding partners which are antibodies chosen to select a particular organism or organisms such that the detectable organisms become selectively bound to the coded beaded supports as a result of the occurrence of specific binding partner interactions;
the method further comprising the step of sorting the different types of coded beaded supports in a suitable particle sorter before determining the presence, absence or number of detectable organisms;
and wherein the result of determining the presence, absence or number of detectable organisms immobilised to each different type of coded beaded support is either (iii) correlated with the particular binding partner immobilised to each particle type to thereby determine the presence, absence or number of each of the different organisms of interest in the sample, or portion thereof; or (iv) correlated with the code for a sample source from which the sample, or portion thereof, was derived to thereby determine the presence, absence or number of each of the different organisms of interest in each different sample, or portion thereof.

11. The method of Claim 10, wherein the probes are independently detectable and are labelled with independently detectable fluorophores.

12. The method of Claim 10, wherein the detectable molecular probe or probes stain all of the different organisms with the same stain and wherein the binding partner is specific for each of the different organisms of interest such that the sorting of the different types of coded beaded supports determines each of the different organisms of interest in the sample, or portion thereof, based solely upon the identity of the different binding partner.

13. The method of Claim 10, wherein the independently detectable molecular probe or probes stain all of the organisms of interest provided that some or all of the different organisms of interest are stained differently and wherein each binding partner associated with each different type of coded beaded support is chosen to select among the same or differently stained organisms such that the sorting of the different types of coded beaded supports when considered in combination with the stain of the organism or organisms bound to each different type of coded beaded support determines each of the different organisms of interest in the sample, or portion thereof.

14. The method of Claim 10, wherein the independently detectable molecular probes stain the organism or organisms of interest differently and wherein each binding partner associated with each different type of coded beaded support is generic to the chosen assay but each different coded beaded support codes for a different sample such that the determination of the stain or stains on each different coded beaded support specifically determines each of the one or more organisms of interest in the sample, or portion thereof, and each different coded beaded support identifies the source of the sample, or portion thereof.

15. A method as claimed in any one of Claims 1 to 5 or 7 to 9, wherein the solid carrier comprises a solid carrier array to which binding partners have been immobilised at unique identifiable locations such that detectable organisms are selectively bound to locations on the array as a result of the occurrence of specific binding partner interactions;
and wherein the result of determining the presence, absence or number of detectable organisms immobilised at the different locations of the array is correlated with the particular binding partner immobilised to each location.

16. The method of Claim 15, wherein the detectable molecular probe or probes stain all of the different organisms with the same stain and wherein the binding partner is specific for each of the different organisms of interest such that the sorting of the organisms on the array resulting from the binding partner interactions occurring at the unique locations is used to thereby determine each of the different organisms of interest in the sample, or portion thereof, based solely upon the identity of the different binding partners of the unique locations.

17. The method of Claim 15, wherein the independently detectable molecular probe or probes stain all of the organisms of interest provided that some or all of the different organisms of interest are stained differently and wherein each binding partner associated with a unique location on the array is chosen to select among the same or differently stained organisms such that the sorting of the organisms on the array resulting from the binding partner interactions occurring at the unique locations, when considered in combination with the stain of the organism or organisms bound to each unique location, is used to determine each of the different organisms of interest in the sample, or portion thereof.

18. A composition comprising:
one or more organisms stained with one or more detectable molecular probes wherein the molecular probe or probes are peptide nucleic acid;
a solid carrier to which is immobilised a binding partner which is an antibody wherein the one or more stained organisms are linked to the solid carrier through the interaction of the organism and its binding partner.

19. The composition of Claim 18, wherein the solid carrier is a solid carrier array comprising antibodies to many different organisms of interest which have each been immobilised at unique indentifiable locations on the array.

20. The composition of Claim 18, wherein the solid carrier is a coded beaded support.

21. The composition of Claim 18, wherein the solid carrier is a microscope slide.

22. A composition as claimed in Claim 18, further comprising a second organism detectably or independently stained with a molecular probe which is peptide nucleic acid; and wherein the solid carrier comprises a mixture of two or more different types of coded beaded supports, wherein to each different type of coded beaded support has been immobilised a different binding partner which is an antibody that is selected to detect a particular organism of interest and wherein the different organisms are selectively bound to the coded beaded supports.

23. A composition as claimed in Claim 18, further comprising a second organism detectably or independently stained with a molecular probe which is peptide nucleic acid; and wherein the solid carrier comprises a solid carrier array to which binding partners which are antibodies have been immobilised at unique identifiable locations such that stained organisms are selectively bound to locations on the array.

## Patentansprüche

1. Verfahren zur Bestimmung und Unterscheidung eines Organismus von Interesse in einer Probe von einem anderen Organismus oder anderer Organismen, wobei das Verfahren umfasst;
das Behandeln der Probe oder eines Anteils davon mit mindestens einer nachweisbaren Molekülsonde, wobei die Molekülsonde oder -sonden
Peptidnukleinsäure sind und ausgewählt sind, so dass entweder:
(i) sowohl der Organismus von Interesse als auch die anderen Organismen sich mit der Molekülsonde in einer Weise, welche nachweisbare Organismen von Interesse und einen zu unterscheidenden nachweisbaren anderen Organismus oder zu unterscheidende Organismen, herstellt, umsetzen; oder
(ii) nur der Organismus von Interesse sich mit der Molekülsonde in einer Weise, welche nur nachweisbare Organismen von Interesse herstellt, umsetzt; und
das Inkontaktbringen der Probe oder eines Anteils davon mit einem festen Trägermaterial, an welchem ein Bindungspartner immobilisiert wurde, welcher ein Antikörper ist, so dass, falls (i) zutrifft, dann der Bindungspartner gewählt wird, der nur mit dem nachweisbaren Organismus von Interesse reaktiv ist, aber nicht mit dem nachweisbaren zu unterscheidenden anderen Organismus oder Organismen, reaktiv ist; aber falls (ii) zutrifft, dann der Bindungspartner gewählt wird, der im allgemeinen mit dem nachweisbaren Organismus von Interesse reaktiv ist, aber auch mit dem anderen zu unterscheidenden Organismus oder zu unterscheidenden Organismen, reaktiv sein kann; und
das Bestimmen der Anwesenheit, Abwesenheit, Position oder Anzahl von nachweisbaren Organismen, welche an dem festen Trägermaterial immobilisiert sind, und das Korrelieren des Ergebnisses mit der Anwesenheit, Abwesenheit oder Anzahl der Organismen von Interesse in der Probe oder einem Anteil davon.

2. Verfahren nach Anspruch 1, wobei die nachweisbare Molekülsonde nicht mit einer nachweisbaren Einheit markiert ist.

3. Verfahren nach Anspruch 2, wobei die nachweisbare Molekülsonde unter Verwendung eines nachweisbaren Antikörpers, welcher spezifisch an einen nachweisbare Molekülsonde/Zielsequenz-Komplex bindet, nachgewiesen wird.

4. Verfahren nach Anspruch 1, wobei die nachweisbare Molekülsonde mit einer nachweisbaren Einheit markiert ist.

5. Verfahren nach Anspruch 4, wobei die nachweisbare Einheit aus der Gruppe ausgewählt ist, welche aus einem Chromophor, einem Fluorochrom, einer Spin-Markierung, einem Radioisotop, einem Enzym, einem Hapten und einer chemilumineszenten Verbindung besteht.

6. Verfahren nach Anspruch 1, wobei das feste Trägermaterial aus der Gruppe ausgewählt ist, welche aus einem Partikel, einem Kügelchen, einem Objektträger, einer Mikrotiterplatte, einer Membran und einem Array besteht.

7. Verfahren nach Anspruch 1, wobei die Probe oder ein Anteil davon mit der bzw. den nachweisbaren Molekülsonde oder -sonden behandelt wird, bevor sie/er mit dem festen Trägermaterial in Kontakt gebracht wird.

8. Verfahren nach Anspruch 1, wobei die Probe oder ein Anteil davon mit dem festen Trägermaterial in Kontakt gebracht wird, bevor sie/er mit der bzw. den nachweisbaren Molekülsonde oder -sonden behandelt wird.

9. Verfahren nach Anspruch 1, wobei die Probe oder ein Anteil davon gleichzeitig sowohl mit dem festen Trägermaterial in Kontakt gebracht als auch mit der bzw. den nachweisbaren Molekülsonde oder -sonden behandelt wird.

10. Verfahren wie in einem der Ansprüche 1 bis 5 oder 7 bis 9 beansprucht, wobei das feste Trägermaterial zwei oder mehr unterschiedliche Typen von kodierten Trägem in Kügelchenform umfasst, wobei jeder unterschiedliche Typ von kodiertem Träger in Kügelchenform unabhängig in einer geeigneten Partikelsortiervorrichtung bestimmt werden kann, wobei an den kodierten Trägem in Kügelchenform ein oder mehrere Bindungspartner immobilisiert wurden, welche Antikörper sind, die ausgewählt wurden, um einen besonderen Organismus oder Organismen zu selektieren, so dass die nachweisbaren Organismen selektiv an die kodierten Träger in Kügelchenform als ein Ergebnis des Auftretens von spezifischen Bindungspartnerwechselwirkungen gebunden werden;
wobei das Verfahren ferner den Schritt des Sortierens der unterschiedlichen Typen von kodierten Trägem in Kügelchenform in einer geeigneten Partikelsortiervorrichtung umfasst, bevor die Anwesenheit, Abwesenheit oder Anzahl von nachweisbaren Organismen bestimmt wird;
und wobei das Ergebnis der Bestimmung der Anwesenheit, Abwesenheit oder Anzahl von nachweisbaren Organismen, welche an jedem unterschiedlichen Typ von kodiertem Träger in Kügelchenform immobilisiert sind, entweder (iii) mit dem besonderen Bindungspartner, welcher an jedem Partikeltyp immobilisiert ist, korreliert wird, um dabei die Anwesenheit, Abwesenheit oder Anzahl von jedem der unterschiedlichen Organismen von Interesse in der Probe oder einem Anteil davon zu bestimmen; oder (iv) mit dem Kode für eine Probenquelle, aus welcher die Probe oder ein Anteil davon abgeleitet wurde, korreliert wird, um dabei die Anwesenheit, Abwesenheit oder Anzahl von jedem der unterschiedlichen Organismen von Interesse in jeder unterschiedlichen Probe oder einem Anteil davon zu bestimmen.

11. Verfahren nach Anspruch 10, wobei die Sonden unabhängig nachweisbar sind und mit unabhängig nachweisbaren Fluorophoren markiert sind.

12. Verfahren nach Anspruch 10, wobei die nachweisbare Molekülsonde oder -sonden alle der unterschiedlichen Organismen mit der gleichen Färbung färben und wobei der Bindungspartner spezifisch für jeden der unterschiedlichen Organismen von Interesse ist, so dass das Sortieren der unterschiedlichen Typen von kodierten Trägem in Kügelchenform jeden der unterschiedlichen Organismen von Interesse in der Probe oder einem Anteil davon beruhend nur auf der Identität des unterschiedlichen Bindungspartners bestimmt.

13. Verfahren nach Anspruch 10, wobei die unabhängig nachweisbare Molekülsonde oder -sonden alle der Organismen von Interesse färben, mit der Maßgabe dass einige oder alle der unterschiedlichen Organismen von Interesse unterschiedlich gefärbt werden, und wobei jeder Bindungspartner, assoziiert mit jedem unterschiedlichen Typ von kodiertem Träger in Kügelchenform, gewählt wird, um unter den gleich oder unterschiedlich gefärbten Organismen zu selektieren, so dass das Sortieren der unterschiedlichen Typen von kodierten Trägem in Kügelchenform, wenn in Kombination mit der Färbung des Organismus oder der Organismen betrachtet, welche an jedem unterschiedlichen Typ von kodiertem Träger in Kügelchenform gebunden sind, jeden der unterschiedlichen Organismen von Interesse in der Probe oder einem Anteil davon bestimmt.

14. Verfahren nach Anspruch 10, wobei die unabhängig nachweisbaren Molekülsonden den Organismus oder die Organismen von Interesse unterschiedlich färben und wobei jeder Bindungspartner, assoziiert mit jedem unterschiedlichen Typ von kodiertem Träger in Kügelchenform, generisch für den gewählten Test ist, aber jeder unterschiedliche kodierte Träger in Kügelchenform für eine unterschiedliche Probe kodiert, so dass die Bestimmung der Färbung oder Färbungen an jedem unterschiedlichen kodierten Träger in Kügelchenform spezifisch jeden des einen oder der mehreren Organismen von Interesse in der Probe oder einem Anteil davon bestimmt und jeder unterschiedliche kodierte Träger in Kügelchenform die Quelle der Probe oder eines Anteils davon identifiziert.

15. Verfahren wie in einem der Ansprüche 1 bis 5 oder 7 bis 9 beansprucht, wobei das feste Trägermaterial einen festen Trägerarray umfasst, an welchem Bindungspartner an speziellen identifizierbaren Orten immobilisiert wurden, so dass nachweisbare Organismen selektiv an Orten auf dem Array als ein Ergebnis des Auftretens von spezifischen Bindungspartnerwechselwirkungen gebunden werden;
und wobei das Ergebnis der Bestimmung der Anwesenheit, Abwesenheit oder Anzahl von nachweisbaren Organismen, welche an den unterschiedlichen Orten des Arrays immobilisiert sind, mit dem besonderen Bindungspartner, welcher an jedem Ort immobilisiert ist, korreliert wird.

16. Verfahren nach Anspruch 15, wobei die nachweisbare Molekülsonde oder -sonden alle der unterschiedlichen Organismen mit der gleichen Färbung färben und wobei der Bindungspartner spezifisch für jeden der unterschiedlichen Organismen von Interesse ist, so dass das Sortieren der Organismen auf dem Array, welches aus den Bindungspartnerwechselwirkungen resultiert, welche an den speziellen Orten auftreten, verwendet wird, um dabei jeden der unterschiedlichen Organismen von Interesse in der Probe oder einem Anteil davon basierend nur auf der Identität der unterschiedlichen Bindungspartner der speziellen Orte zu bestimmen.

17. Verfahren nach Anspruch 15, wobei die unabhängig nachweisbare Molekülsonde oder -sonden alle der Organismen von Interesse färben, mit der Maßgabe dass einige oder alle der unterschiedlichen Organismen von Interesse unterschiedlich gefärbt werden, und wobei jeder Bindungspartner, assoziiert mit einem speziellen Ort auf dem Array, gewählt wird, um unter den gleich oder unterschiedlich gefärbten Organismen zu selektieren, so dass das Sortieren der Organismen auf dem Array, welches aus den Bindungspartnerwechselwirkungen resultiert, welche an den speziellen Orten auftreten, wenn in Kombination mit der Färbung des Organismus oder der Organismen betrachtet, welche an jedem speziellen Ort gebunden sind, verwendet wird, um jeden der unterschiedlichen Organismen von Interesse in der Probe oder einem Anteil davon zu bestimmen.

18. Zusammensetzung, umfassend:
einen oder mehrere Organismen, welche mit einer oder mehreren nachweisbaren Molekülsonden gefärbt sind, wobei die Molekülsonde oder -sonden Peptidnukleinsäure sind;
ein festes Trägermaterial, an welchem ein Bindungspartner immobilisiert ist, welcher ein Antikörper ist, wobei der eine oder die mehreren gefärbten Organismen an das feste Trägermaterial durch die Wechselwirkung des Organismus und seines Bindungspartners gebunden sind.

19. Zusammensetzung nach Anspruch 18, wobei das feste Trägermaterial ein fester Trägerarray ist, welcher Antikörper für viele unterschiedliche Organismen von Interesse umfasst, welche jeweils an speziellen identifizierbaren Orten auf dem Array immobilisiert wurden.

20. Zusammensetzung nach Anspruch 18, wobei das feste Trägermaterial ein kodierter Träger in Kügelchenform ist.

21. Zusammensetzung nach Anspruch 18, wobei das feste Trägermaterial ein Objektträger ist.

22. Zusammensetzung wie in Anspruch 18 beansprucht, ferner umfassend einen zweiten Organismus, welcher nachweisbar oder unabhängig mit einer Molekülsonde gefärbt ist, welche eine Peptidnukleinsäure ist;
und wobei das feste Trägermaterial ein Gemisch von zwei oder mehreren unterschiedlichen Typen von kodierten Trägern in Kügelchenform umfasst, wobei an jedem unterschiedlichen Typ von kodiertem Träger in Kügelchenform ein unterschiedlicher Bindungspartner immobilisiert wurde, welcher ein Antikörper ist, der ausgewählt ist, einen besonderen Organismus von Interesse nachzuweisen, und
wobei die unterschiedlichen Organismen selektiv an die kodierten Träger in Kügelchenform gebunden sind.

23. Zusammensetzung wie in Anspruch 18 beansprucht, ferner umfassend einen zweiten Organismus, welcher nachweisbar oder unabhängig mit einer Molekülsonde gefärbt ist, welche eine Peptidnukleinsäure ist;
und wobei das feste Trägermaterial einen festen Trägerarray umfasst, an welchem Bindungspartner, welche Antikörper sind, an speziellen identifizierbaren Orten immobilisiert wurden, so dass gefärbte Organismen selektiv an Orten auf dem Array gebunden werden.

## Revendications

1. Procédé de détermination d'un organisme d'intérêt dans un échantillon par rapport à un autre organisme ou à d'autres organismes à distinguer, ledit procédé comprenant les étapes consistant à :
traiter l'échantillon, ou une partie de celui-ci, avec au moins une sonde moléculaire détectable dans lequel la sonde ou les sondes moléculaires sont un acide nucléique peptidique et sont choisies de telle sorte que soit :
(i) l'organisme d'intérêt et les autres organismes réagissent tous avec la sonde moléculaire d'une manière qui produit des organismes d'intérêt détectables et un ou plusieurs autres organismes détectables à distinguer ; soit
(ii) seul l'organisme d'intérêt réagit avec la sonde moléculaire d'une manière qui produit uniquement des organismes d'intérêt détectables ; et
mettre en contact l'échantillon, ou une partie de celui-ci, avec un support solide sur lequel a été immobilisé un partenaire de liaison qui est un anticorps de telle sorte que, si (i) s'applique, alors le partenaire de liaison est choisi pour être réactif uniquement avec l'organisme d'intérêt détectable mais ne pas être réactif avec l'autre organisme ou les autres organismes détectables à distinguer ; mais si (ii) s'applique, alors le partenaire de liaison est choisi pour être généralement réactif avec l'organisme d'intérêt détectable mais il peut aussi être réactif avec l'autre organisme ou les autres organismes à distinguer ; et
déterminer la présence, l'absence, la position ou le nombre d'organismes détectables immobilisés sur le support solide et corréler le résultat avec la présence, l'absence ou le nombre des organismes d'intérêt dans l'échantillon, ou une partie de celui-ci.

2. Procédé selon la revendication 1, dans lequel la sonde moléculaire détectable n'est pas marquée avec un fragment détectable.

3. Procédé selon la revendication 2, dans lequel la sonde moléculaire détectable est détectée en utilisant un anticorps détectable qui se lie spécifiquement à une sonde moléculaire/un complexe de séquences cibles détectable.

4. Procédé selon la revendication 1, dans lequel la sonde moléculaire détectable est marquée avec un fragment détectable.

5. Procédé selon la revendication 4, dans lequel le fragment détectable est choisi dans le groupe constitué par : un chromophore, un fluorochrome, un marqueur de spin, un radio-isotope, une enzyme, un haptène et un composé à chimiluminescence.

6. Procédé selon la revendication 1, dans lequel le support solide est choisi dans le groupe constitué par : une particule, une perle, une lame pour microscope, une microplaque à titration, une membrane et une puce.

7. Procédé selon la revendication 1, dans lequel l'échantillon, ou une partie de celui-ci, est traité avec la sonde ou les sondes moléculaires détectables avant d'être mis en contact avec le support solide.

8. Procédé selon la revendication 1, dans lequel l'échantillon, ou une partie de celui-ci, est mis en contact avec le support solide avant d'être traité avec la sonde ou les sondes moléculaires détectables.

9. Procédé selon la revendication 1, dans lequel l'échantillon, ou une partie de celui-ci, est simultanément mis en contact avec le support solide et traité avec la sonde ou les sondes moléculaires détectables.

10. Procédé selon l'une quelconque des revendications 1 à 5 ou 7 à 9, dans lequel le support solide comprend deux ou davantage de types différents de supports perlés codés, dans lequel chaque type différent de support perlé codé peut être déterminé indépendamment dans un trieur de particules approprié, dans lequel sur les supports perlés codés ont été immobilisés un ou plusieurs partenaires de liaison qui sont des anticorps choisis pour sélectionner un organisme ou des organismes particuliers de telle sorte que les organismes détectables se trouvent sélectivement liés aux supports perlés codés en résultat de l'existence d'interactions entre partenaires de liaison spécifiques ;
le procédé comprenant en outre l'étape consistant à trier les différents types de supports perlés codés dans un trieur de particules approprié avant de déterminer la présence, l'absence ou le nombre d'organismes détectables ;
et dans lequel le résultat de la détermination de la présence, de l'absence ou du nombre d'organismes détectables immobilisés sur chaque type différent de support perlé codé est soit (iii) corrélé avec le partenaire de liaison particulier immobilisé sur chaque type de particules pour déterminer ainsi la présence, l'absence ou le nombre de chacun des différents organismes d'intérêt dans l'échantillon, ou une partie de celui-ci ; soit (iv) corrélé avec le code pour une source d'échantillon à partir de laquelle l'échantillon, ou une partie de celui-ci, a été dérivé pour déterminer ainsi la présence, l'absence ou le nombre de chacun des différents organismes d'intérêt dans chaque échantillon différent, ou une partie de celui-ci.

11. Procédé selon la revendication 10, dans lequel les sondes sont indépendamment détectables et sont marquées avec des fluorophores indépendamment détectables.

12. Procédé selon la revendication 10, dans lequel la sonde ou les sondes moléculaires détectables colorent tous les organismes différents avec la même coloration et dans lequel le partenaire de liaison est spécifique de chacun des différents organismes d'intérêt de telle sorte que le tri des types différents de supports perlés codés détermine chacun des différents organismes d'intérêt dans l'échantillon, ou une partie de celui-ci, basé exclusivement sur l'identité des différents partenaires de liaison.

13. Procédé selon la revendication 10, dans lequel la sonde ou les sondes moléculaires indépendamment détectables colorent tous les organismes d'intérêt à condition que quelques-uns ou tous les différents organismes d'intérêt soient colorés différemment et dans lequel chaque partenaire de liaison associé à chaque type différent de support perlé codé est choisi pour sélectionner parmi les organismes colorés de manière identique ou les organismes colorés différemment de telle sorte que le tri des types différents de supports perlés codés, lorsque considéré en combinaison à la coloration de l'organisme ou des organismes liés à chaque type différent de support perlé codé, détermine chacun des différents organismes d'intérêt dans l'échantillon, ou une partie de celui-ci.

14. Procédé selon la revendication 10, dans lequel les sondes moléculaires indépendamment détectables colorent différemment l'organisme ou les organismes d'intérêt et dans lequel chaque partenaire de liaison associé à chaque type différent de support perlé codé est générique pour le dosage choisi mais chaque support perlé codé différent code pour un échantillon différent de telle sorte que la détermination de la coloration ou des colorations sur chaque support perlé codé différent détermine spécifiquement chacun desdits un ou plusieurs organismes d'intérêt dans l'échantillon, ou une partie de celui-ci, et chaque support perlé codé différent identifie la source de l'échantillon, ou d'une partie de celui-ci.

15. Procédé selon l'une quelconque des revendications 1 à 5 ou 7 à 9, dans lequel le support solide comprend un support solide de puce sur lequel des partenaires de liaison ont été immobilisés en des emplacements identifiables uniques de telle sorte que les organismes détectables soient sélectivement liés aux emplacements sur la puce en résultat de l'existence d'interactions entre partenaires de liaison spécifiques ;
et dans lequel le résultat de la détermination de la présence, de l'absence ou du nombre d'organismes détectables immobilisés aux différents emplacements de la puce est corrélé avec le partenaire de liaison particulier immobilisé sur chaque emplacement.

16. Procédé selon la revendication 15, dans lequel la sonde ou les sondes moléculaires détectables colorent tous les organismes différents avec la même coloration et dans lequel le partenaire de liaison est spécifique de chacun des différents organismes d'intérêt de telle sorte que le tri des organismes sur la puce résultant des interactions entre partenaires de liaison existant aux emplacements uniques soit utilisé pour déterminer ainsi chacun des différents organismes d'intérêt dans l'échantillon, ou une partie de celui-ci, basé exclusivement sur l'identité des différents partenaires de liaison des emplacements uniques.

17. Procédé selon la revendication 15, dans lequel la sonde ou les sondes moléculaires indépendamment détectables colorent tous les organismes d'intérêt à condition que quelques-uns ou tous les différents organismes d'intérêt soient colorés différemment et dans lequel chaque partenaire de liaison associé à un emplacement unique sur la puce est choisi pour sélectionner parmi les organismes colorés de manière identique ou les organismes colorés différemment de telle sorte que le tri des organismes sur la puce résultant des interactions entre partenaires de liaison existant aux emplacements uniques, lorsque considéré en combinaison à la coloration de l'organisme ou des organismes liés à chaque emplacement unique, soit utilisé pour déterminer chacun des différents organismes d'intérêt dans l'échantillon, ou une partie de celui-ci.

18. Composition comprenant :
un ou plusieurs organismes colorés avec une ou plusieurs sondes moléculaires détectables dans laquelle la sonde ou les sondes moléculaires sont un acide nucléique peptidique ;
un support solide sur lequel est immobilisé un partenaire de liaison qui est un anticorps dans laquelle lesdits un ou plusieurs organismes colorés sont liés au support solide par l'interaction de l'organisme et de son partenaire de liaison.

19. Composition selon la revendication 18, dans laquelle le support solide est un support solide de puce comprenant des anticorps contre de nombreux organismes d'intérêt différents qui ont été immobilisés en des emplacements identifiables uniques sur la puce.

20. Composition selon la revendication 18, dans laquelle le support solide est un support perlé codé.

21. Composition selon la revendication 18, dans laquelle le support solide est une lame pour microscope.

22. Composition selon la revendication 18, comprenant en outre un deuxième organisme coloré de manière détectable ou de manière indépendante avec une sonde moléculaire qui est un acide nucléique peptidique ;
et dans laquelle le support solide comprend un mélange de deux ou davantage de types différents de supports perlés codés, dans laquelle sur chaque type différent de support perlé codé a été immobilisé un partenaire de liaison différent qui est un anticorps qui est choisi pour détecter un organisme d'intérêt particulier et dans laquelle les organismes différents sont sélectivement liés aux supports perlés codés.

23. Composition selon la revendication 18, comprenant en outre un deuxième organisme coloré de manière détectable ou de manière indépendante avec une sonde moléculaire qui est un acide nucléique peptidique ;
et dans laquelle le support solide comprend un support solide de puce sur lequel des partenaires de liaison qui sont des anticorps ont été immobilisés en des emplacements identifiables uniques de telle sorte que les organismes colorés soient sélectivement liés aux emplacements sur la puce.


### SEQUENCE LISTING

<110> Boston Probes, Inc.
<120> Methods And Compositions For Sorting And/Or Determining Organisms
<130> BP0002-PCT
<140>
   <141>
<150> 60/250,930 <151> 2000-11-30
<160> 1
<170> PatentIn Ver. 2.1
<210> 1
   <211> 15
   <212> DNA
   <213> Artificial Sequence
<220>
   <223> Description of Combined DNA/RNA Molecule: Artificial PNA Probe Sequence
<220>
   <221> misc_feature
   <222> (1)
   <223> N-Terminal Fluorescein
<220>
   <223> Description of Artificial Sequence: Artificial PNA Probe
<400> 1
   ctgcctcccg tagga 15